Europäisches Patentamt

European Patent Office

Office européen des brevets

(11) Veröffentlichungsnummer: **0 319 879**
**A2**

(19)

(12) **EUROPÄISCHE PATENTANMELDUNG**

(21) Anmeldenummer: 88120231.1

(22) Anmeldetag: 03.12.88

(51) Int. Cl.⁴: **A61K 35/78**

(30) Priorität: 11.12.87 DE 3741994

(43) Veröffentlichungstag der Anmeldung:
14.06.89 Patentblatt 89/24

(84) Benannte Vertragsstaaten:
AT BE CH DE ES FR GB GR IT LI LU NL SE

(71) Anmelder: Nini, Yves
Am Schulterblatt 98
D-2000 Hamburg 6(DE)

(72) Erfinder: Nini, Yves
Am Schulterblatt 98
D-2000 Hamburg 6(DE)

(74) Vertreter: Keil, Rainer A., Dipl.-Phys. Dr. et al
KEIL & SCHAAFHAUSEN Patentanwälte
Eysseneckstrasse 31
D-6000 Frankfurt am Main 1(DE)

(54) **Extrakte des Judasbaumes als prophylaktische und/oder therapeutische Wirkstoffe.**

(57) Die Erfindung bezieht sich auf Extrakte des Judasbaumes zur Anwendung als prophylaktische und/oder therapeutische Wirkstoffe sowie als Mittel zur Dekontamination radioaktiv kontaminierter "toter" Materie.

EP 0 319 879 A2

## Extrakte des Judasbaumes als prophylaktische und/oder therapeutische Wirkstoffe

Die Erfindung betrifft ein Mittel zur Dekorporation eines radioaktiv kontaminierten menschlichen oder tierischen Körpers sowie ein Mittel zur Dekontamination radioaktiv kontaminierter Materie.

Die gesundheitsschädliche Wirkung von radioaktiver Strahlung auf den menschlichen oder tierischen Körper, insbesondere diejenige im Körper abgelagerter (inkorporierter) radioaktiver Substanzen, ist hinlänglich bekannt. Bei einer Inkorporation radioaktiver Nuklide im Körper, welche beispielsweise durch Störfälle bei Kernreaktoren, Kernwaffenexplosionen oder dgl. in die Atmosphäre emittiert werden und als radioaktiver Niederschlag durch die Nahrungsmittelkette oder über die Atemluft in den menschlichen oder tierischen Körper gelangen können, ist der Körper einer ständigen Strahlenbelastung ausgesetzt, bis die Radioaktivität aufgrund der physikalischen Halbwertzeit der Nuklide abgeklungen bzw. die Nuklide aufgrund der biologischen Halbwertzeit wieder ausgeschieden werden. Ähnliche Probleme treten bei in Kernreaktoranlagen oder radioaktiven Labors beschäftigten Personen auf. Die Radionuklide können große physikalische und biologische Halbwertzeiten aufweisen. Übermäßige Strahlenbelastung führt zu somatischen und genetischen Schädigungen. Bisher wurde noch kein Mittel gefunden, das geeignet ist, radioaktive Substanzen im Körper einer kontaminierten Person oder eines kontaminierten Tieres beschleunigt abzubauen oder gar als Prophylaktikum für gefährdete Personen oder Tiere zu wirken. Ebenso wäre es wünschenswert, wenn ein Mittel zur Verfügung stünde, welches einen beschleunigten Abbau der Radioaktivität von radioaktiv kontaminierter "toter" Materie, wie bspw. radioaktiv kontaminierter Abfall von Kernkraftwerken o. dgl., herbeiführen würde.

Aufgabe der vorliegenden Erfindung ist es, ein einfach herstellbares Mittel zu finden, welches ein schnelles Ausscheiden der radioaktiven Nuklide aus dem menschlichen oder tierischen Körper bewirkt und gleichzeitig als Prophylaktikum dient und ebenso einen schnellen Abbau der Radioaktivität von radioaktiv kontaminierter "toter" Materie erzeugt.

Es wurde nun gefunden, daß Extrakte des Judasbaumes als prophylaktische und/oder therapeutische Wirkstoffe, insbesondere als eine Inkorporation radioaktiver Substanzen des menschlichen oder tierischen Körpers abbauende Wirkstoffe angewendet werden können.

Völlig überraschend hat sich bei Versuchen gezeigt, daß durch Einnahme dieser Extrakte die Menge der radioaktoven Substanzen, insbesondere wie sie bei Kernreaktorstörfällen frei werden und von Menschen und Tieren inkorporiert worden sind, bereits nach wenigen Tagen erheblich gesenkt werden konnte. Offenbar lagern sich die im Körper enthaltenen radioaktiven Nuklide bevorzugt an den Extrakten des Judasbaumes an, vom Verständnis her offenbar ähnlich, wie dies für die Isotopen des Jods bei der Schilddrüse der Fall ist. Durch die Ausscheidung der mit den radioaktiven Nukliden angereicherten Extrakte ergibt sich der überraschende Abbau der Radioaktivität im Körper.

Die Extrakte können beispielsweise durch Essen oder Verfüttern von Teilen des Judasbaumes in den menschlichen oder tierischen Körper gelangen und ihre Wirkung entfalten. Besondere Vorteile erreicht man jedoch, wenn die Extrakte durch Mahlen oder Zerreiben der Bestandteile des Judasbaumes gewonnen sind, wodurch die Wirksamkeit für den Abbau der radioaktiven Nuklide verbessert wird.

Neben der Verwendung aller Bestandteile des Baumes können die Extrakte auch durch Mahlen eines Bestandteiles des Judasbaumes, beispielsweise der Wurzeln, des Stammes, der Rinde oder bevorzugt der Blätter, ausgenommen der Blüten, gewonnen sein.

Als Darreichungsform können die Extrakte ggf. nach dem Mahlen in Tablettenform gebracht sein.

Alternativ ist es auch möglich, daß sie in einer durch die Magen-und/oder Darmsäfte auflösbaren Kapsel enthalten sind, wodurch u.a. im Hinblick auf eine Prophylaxe eine gewisse Retardwirkung erreicht ist.

Völlig überraschend hat sich auch gezeigt, daß die Extrakte des Judasbaumes zur Dekontamination radioaktiv kontaminierter Stoffe oder Substanzen, wie bspw. radioaktiver Erde, Baumaterialien o. dgl. eingesetzt werden können. So konnte im Falle von radioaktiv kontaminiertem Thymian durch Zugabe der Extrakte des Judasbaumes ein beschleunigter Abbau der Radioaktivität bereits nach weniger als einem Tag nachgewiesen werden.

Auch hierbei ist es besonders günstig, wenn die Extrakte durch Mahlen oder Zerreiben der Bestanteile des Judasbaumes gewonnen sind, wodurch sich die Wirksamkeit des Abbaus der Radioaktivität erhöht.

Die Extrakte können durch Mahlen oder Zerreiben aller oder einzelner Bestandteile des Baumes, bspw. der Wurzeln, des Stammes, der Rinde oder bevorzugt der Blätter, ausgenommen der Blüten, gewonnen sein.

Die Wirksamkeit der Extrakte läßt sich noch dadurch steigern, daß sie angefeuchtet sind, bspw. einen, abhängig von dem Feuchtegrad der Extrakte selbst, vorgegebenen Bestandteil an Wasser aufweisen.

In die gleiche Richtung zielt auch der erfindungsgemäße Vorschlag, daß die Extrakte eine Beimischung von Kupfer und/oder Silber aufweisen, von welchen offenbar eine katalytische Wirkung in Bezug auf einen beschleunigten Abbau der Radioaktivität von kontaminierten Stoffen und Substanzen ausgeht.

Selbstverständlich liegt es auch im Rahmen des Erfindungsgedankens, die gleichen Wirkungen durch einen demjenigen der Extrakte des Judasbaumes vergleichbaren synthetisch hergestellten Wirkstoff bzw. einen synthetisch hergestellten Stoff zu erzielen.

**Ansprüche**

1. Extrakte des Judasbaumes (Cerxis siliquastrum L.) zur Anwendung als prophylaktische und/oder therapeutische Wirkstoffe.

2. Extrakte nach Anspruch 1 zur Anwendung als eine radioaktive Inkorporation radioaktiver Substanzen im menschlichen oder tierischen Körper abbauende Wirkstoffe.

3. Extrakte nach Anspruch 1 oder 2, dadurch gekennzeichnet, daß sie durch Mahlen der Bestandteile des Judasbaumes gewonnen sind.

4. Extrakte nach einem der Ansprüche 1 bis 3, dadurch gekennzeichnet, daß sie durch Mahlen der Wurzeln, des Stammes, der Rinde und/oder der Blätter des Judasbaumes gewonnen sind.

5. Extrakte nach einem der Ansprüche 1 bis 4, dadurch gekennzeichnet, daß sie, ggf. nach dem Mahlen, in Tablettenform gebracht sind.

6. Extrakte nach einem der Ansprüche 1 bis 4, dadurch gekennzeichnet, daß sie in einer durch die Magen- und/oder Darmsäfte auflösbaren Kapsel enthalten sind.

7. Extrakte des Judasbaumes zur Dekontamination radioaktiv kontaminierter Stoffe oder Substanzen, wie bspw. radioaktive Abfälle, Erde, Baumaterialien o. dgl.

8. Extrakte nach Anspruch 7, dadurch gekennzeichnet, daß sie durch Mahlen der Bestandteile des Judasbaumes gewonnen sind.

9. Extrakte nach Anspruch 7 oder 8, dadurch gekennzeichnet, daß sie durch Mahlen der Wurzeln, des Stammes, der Rinde und/oder der Blätter des Judasbaumes gewonnen sind.

10. Extrakte nach einem der Ansprüche 7 bis 9, dadurch gekennzeichnet, daß sie angefeuchtet sind.

11. Extrakte nach einem der Ansprüche 1 bis 10, dadurch gekennzeichnet, daß sie eine Beimischung von Kupfer und/oder Silber aufweisen.